# EUROPÄISCHE PATENTSCHRIFT

(11) **EP 3 209 352 B1**
(45) Veröffentlichungstag und Bekanntmachung des Hinweises auf die Patenterteilung: **08.12.2021**
(21) Anmeldenummer: 15797261.3
(22) Anmeldetag: 22.10.2015
(51) Int. Cl.: A61M 5/20, A61M 5/30

(54) **NADELLOSE INJEKTIONSVORRICHTUNG AUFWEISEND EIN GEL UND EINE MEMBRAN**
NEEDLE-FREE INJECTION DEVICE WITH A GEL AND A MEMBRANE
DISPOSITIF D'INJECTION SANS AIGUILLE DOTÉ D'UN GEL ET D'UNE MEMBRANE

(30) Priorität: 22.10.2014 EP 14189971
(43) Veröffentlichungstag der Anmeldung: 30.08.2017
(73) Patentinhaber: Lell, Peter, 85368 Moosburg (DE)
(72) Erfinder: LELL, Peter, 85368 Moosburg (DE); ENGERT, Julia, 80993 München (DE); ANAMUR, Cihad, 67059 Ludwigshafen (DE); FELLNER, Christian, 85304 Ilmmünster (DE); WINTER, Gerhard, 82377 Penzberg (DE)
(74) Vertreter: Simandi, Claus
(86) Internationale Anmeldenummer: PCT/EP2015/074534
(87) Internationale Veröffentlichungsnummer: WO 2016/062825

(56) Entgegenhaltungen:
- WO-A1-00/03758
- WO-A1-2004/071558
- WO-A2-98/31409
- FR-A1- 2 875 413
- US-A1- 2003 114 789

## Beschreibung

Die Erfindung betrifft eine nadellose Injektionsvorrichtung, umfassend eine Brennkammer zur nadellosen Injektion einer Substanz, aufweisend eine Brennkammer, enthaltend ein pyrotechnisches Material als auch einen gelartigen Stoff, mit der mittels einer Membran eine Substanz, insbesondere ein Wirkstoff, auf hohe Geschwindigkeit beschleunigt und in ein Gewebe oder in einen Körper nadellos injiziert werden kann. Weiterhin betrifft die Offenbarung ein Verfahren zur Herstellung einer solchen nadellosen Injektionsvorrichtung enthaltend eine Brennkammer samt pyrotechnischen Material und gefüllt mit einem gelartigen Stoff und dessen Verwendung.

Eine erfolgreiche Injektion besteht darin, dass genügend Substanz durch die Gewebe-, oder Körperbarriere, insbesondere solche wie die Haut von Menschen oder Tieren, in den Körper eingebracht wird. Das Grundprinzip, einen Stoff nadellos mittels hohen Drucks zu injizieren, ist seit geraumer Zeit bekannt (siehe z. B. US 3,308,818).

Es sind verschiedene Typen von nadellosen Injektionsvorrichtungen im Stand der Technik beschrieben, wie z. B. nicht abschließend eine Zylinder-Kolben-Einheit umfassend ein vorgespanntes Federelement (DE 10 2007 004 211 A1) oder mittels sogenannter Injektionspatronen (WO 98/31409), als auch mittels einer Gaspatrone zur Beaufschlagung eines Kolbens (US 5,399,163).

Weiterhin können nadellose Injektionsvorrichtungen einen pyrotechnischen Antrieb enthalten, der beispielsweise zur Betätigung eines Kolbens genutzt wird und auf diese Weise das Auspressen eines Wirkstoffes aus einer Kanüle erzeugt (EP 1 557 190 A1). Eine andere nadellose Injektionsvorrichtung welche auf einem pyrotechnischen Antrieb basiert ist aus US 2003/114789 bekannt.

Eine weitere Ausführungsform einer nadellosen pyrotechnisch betriebenen Injektionsvorrichtung besteht darin, daß in einer Kammer (auch Brennkammer genannt) eine Explosion erzeugt wird, wobei die freigesetzte Energie auf eine Membran so einwirkt, daß eine membranständige, außen in Richtung des Zielgewebes adsorbierte Substanz unter Ablösung von der Membran in Richtung des Zielgewebes hinreichend hoch beschleunigt wird.

Eine solche Vorrichtung ist zum Beispiel in WO 2004/071558 A1 offenbart. Es gilt jedoch eine solche gattungsmäßige Ausführungsform zu verbessern, so dass eine verbesserte Einbringung einer gewünschten Substanz in einen Körper erfolgen kann.

Von daher nimmt die vorliegende Erfindung Bezug auf eine gattungsmäßige Ausführungsform gemäß WO 2004/071558, jedoch mit der objektiven Aufgabenstellung, die nadelfreie Injektion zu verbessern.

Daher betrifft die vorliegende Erfindung eine nadellose Injektionsvorrichtung umfassend eine (Brenn)-Kammer (11) aufweisend a) mindestens ein pyrotechnisches Material (24a) und b) mindestens einen gelartigen Stoff (24b), wobei die Kammer (11) an einer Austrittsöffnung mindestens eine Membran (15) aufweist, und mit einer Substanzauftragung (25) versehen ist.

Die brennkammerseitige Membran (13) mündet zur Brennkammer (11) und dessen Verbrennungsraum (24).

Im Rahmen dieser Erfindung wird unter einem "gelartigen Stoff" oder einem "Gel" eine Mischung von mindestens einer Flüssigkeit (Lyogel) mit mindestens einem Gelbildner verstanden, die zu einer Steigerung der Viskosität der ansonsten weniger viskosen Flüssigkeit führen. Bevorzugte Flüssigkeiten sind erfindungsgemäß hydrophobe Flüssigkeiten. Weiterhin bevorzugt liegt der gelartige Stoff in einer Phase vor. Zudem ist der gelartige Soff von dem pyrotechnischen Material chemisch verschieden. Der gelartige Stoff ist mit dem pyrotechnischen Material nicht identisch oder gleich und hat eine vom pyrotechnischen Material verschiedene Viskosität.

Erfindungsgemäß sind verschiedene Flüssigkeiten sowie verschiedene Gelbildner möglich. Bevorzugt sind jedoch hydrophobe Flüssigkeiten, solche nicht abschließend wie ölige Flüssigkeiten wie pflanzliche oder tierische Öle, Triglyceride, Mono und Diglyceride, Phospholipide, flüssige Wachse, Alkohole, Silikonoele, Paraffine und andere wenig flüchtige hydrophobe Stoffe. Denkbar sind feste und halbfeste und flüssige Gelbildner anorganischer und organischer Natur. Die Gelbildner können hydrophiler, amphiphiler und lipophiler Natur sein. Solche Gelbildner können erfindungsgemäß nicht abschließend sein: Pektin, Tragant, Polyacrylsäuren, Polyvinylpyrrolidon, Siliciumdioxid, hochdisperses Siliciumdioxid, Carboxymethylcellulose, Carbomere (Polyacrylsäure), Celluloseether, Poloxamere (Gele, viskose Lösungen). Die resultierenden gelartigen Stoffe können beispielsweise und vorzugsweise als Oleogele, Lipogele, Silikonoelgele, Paraffingele vorliegen.

Bevorzugt können flüssige, inerte hydrophobe Flüssigkeiten wie Öle (Glyceride) und Silikonöle zusammen mit festen Gelbildnern eingesetzt werden.

Überraschenderweise wurde gefunden, dass mit einem Gelbildner verdickte hydrophobe Flüssigkeiten nicht nur einfacher und sicherer in die Brennkammer eindosiert werden können, sondern zugleich die Funktion der Vorrichtung und deren Sicherheit verbessert werden. Überraschenderweise wurde weiterhin festgestellt, dass insbesondere Silikonoelgele, die mit hydrophilem Siliziumdioxid verdickt sind, besonders vorteilhafte Kombinationen aus Handhabung bei der Herstellung und Funktionalität der Vorrichtung beim bestimmungsgemäßen Gebrauch ermöglichen. Der erfindungsgemäß eingesetzte gelartige Stoff bewirkt vorteilhaft eine Optimierung der Stoß- oder Druckwelle an die Membran und erlaubt gleichzeitig die benötigte Menge an pyrotechnischer Masse (24a) zu minimieren.

Ganz besonders bevorzugt für die Gelierung (Eindickung) von hydrophoben Flüssigkeiten ist daher die Verwendung von Siliziumdioxid insbesondere hochdisperses Siliziumdioxid (z.B. Aerosil R200 Pharma, Aerosil R972 Pharma (Evonik, Deutschland)), vorzugsweise in Verbindung mit einer viskosen Flüssigkeit, wie Silikonöl. Weiterhin ist bevorzugt, dass nach der Verdickung ein Oleogel vorliegt, insbesondere ein Silikonoleogel.

Bevorzugt sind daher weiterhin Gele, die aus Silikonoel und hochdispersem Siliziumdioxid bestehen. Besonders bevorzugt sind Gele, die aus hydrophilem hochdispersem Siliziumdioxid und Silikonoel hergestellt werden.

Das resultierende Gel kann dünnflüssig, dickflüssig oder fest sein. Bevorzugt ist eine Viskosität, bei der die Herstellung der erfindungsgemäßen Vorrichtung vorteilhaft erfolgen kann. Dabei ist das wohldosierte Einbringen in die Brennkammer zu beachten. In einer Ausführungsform ist das Gel dabei dünnflüssig und wird beispielsweise mittels eines Gießverfahrens in die Kammer eindosiert. In einer anderen Ausführungsform ist das Gel dickflüssig und wird mit einem Gieß oder Tropfverfahren in die Kammer eindosiert, eingestrichen oder anderweitig eingebracht. In einer weiteren Ausführungsform ist das Gel halbfest bis fest aber elastisch und wird in einem Stück in die Kammer eingesetzt, eingelegt oder anderweitig eingebracht.

Dabei kann das Gel in einer Portion in vielen kleinen Portionen oder in wenigen größeren Portionen eingebracht werden.

Besonders bevorzugt ist, dass das Leervolumen der Brennkammer so weit wie möglich mit dem erfindungsgemäßen gelartigen Stoff, vorzugsweise mit einer viskosen oder zähflüssigen hydrophoben Masse ausgefüllt ist, so dass eine optimale Ankopplung der von der pyrotechnischen Masse ausgehenden Stoß- oder Druckwelle an die Membran bewirkt wird und gleichzeitig die hierfür benötigte Menge an pyrotechnischer Masse minimiert werden kann.

Dabei wird vorteilhaft das gesamte Volumen der Brennkammer von dem erfindungsgemäßen gelartigen Stoff eingenommen, so dass keine oder nur geringe Gasräume vor der Zündung der pyrotechnischen Masse verbleiben.

Dabei ist es vorteilhaft, dass der gelartige Stoff selbst keine - also gasfrei ist oder nur geringe gasförmige Einschlüsse oder Verunreinigungen enthält.

Weiterhin ist es vorteilhaft, dass bei der Herstellung der gelartigen Stoffe Verfahren angewandt werden, die die Anwesenheit gasförmiger Einschlüsse verhindern oder reduzieren oder einen gasfreies gelartigen Stoff bereitstellen.

Zudem ist es vorteilhaft, bei der Herstellung der gelartigen Stoffe, insbesondere bei der Vorbereitung, der Gelbildner Verfahren anzuwenden, die den Eintrag gasförmiger Einschlüsse minimieren.

Daher betrifft die Erfindung in einer weiteren Ausführungsform eine erfindungsgemäße nadellose Injektionsvorrichtung, wobei die Kammer (11) vollständig mit einem gelartigen Stoff (24b) befüllt ist.

Daher betrifft die Offenbarung ein Verfahren zur Herstellung einer erfindungsgemäßen nadellosen Injektionsvorrichtung, wobei die (Brenn-)Kammer (11) mit einem gelartigen Stoff (24b) bestückt, insbesondere die (Brenn-)Kammer (11) mit einem gelartigen Stoff (24b) vollständig befüllt wird.

Bei der Herstellung erfindungsgemäßer gelartiger Stoffe, wie z.B. Silikonoleogelen werden Verfahren angewandt, die z.B. durch Anwendung von Vakuum den Eintrag von Gas in die gelartigen Stoffe minimieren. Derartige Verfahren sind sowohl im kleinen Maßstab durchführbar (siehe Beispiel 1) als auch in großem Maßstab in entsprechenden Prozessanlagen durchführbar. Im Rahmen dieser Erfindung wird unter "pyrotechnisches Material" ein jedes Material verstanden, welches mit einer Aktivierungsenergie zur Explosion gebracht werden kann. Dies können z.B. feste oder gasförmige Stoffe, wie Azide, Tetrazen, Nitrozellulose, Pikrinsäure, etc. oder andere dem Fachmann bekannte pyrotechnische Materialien sein. Erfindungswesentlich ist, dass die Explosionsenergie eine hinreichend schnelle Ausbauchung der Membran bis hin zu einer ausreichenden Impulsübertragung auf die erfindungsgemäße Membran (15) erlaubt, so dass die Substanzauftragung (25) auf hohe Geschwindigkeiten gebracht werden kann und letztlich die Substanz abgelöst wird.

Das pyrotechnische Material ist erfindungsgemäß insbesondere in einer nach außen vollständig geschlossenen Kammer (11) enthalten und zwar im Verbrennungsraum (24), vorzugsweise in Form eines Detonators (10). Zusätzlich kann weiteres oder anderes pyrotechnisches Material mit eingebracht sein.

Wesentlich ist, dass eine hinreichend schnelle Ausbauchung der Membran oder eine ausreichend große Impulsübertragung auf eine Membran (15), die auch als Doppelmembran bzw. Mehrschichtmembran ausgebildet sein kann, erfolgt, so dass die vor der Auslösung der Vorrichtung außen in Richtung Zielgewebe anhaftenden Partikel/Substanz aus der Substanzauftragung (25) hinreichend beschleunigt werden und zwar vorzugsweise auf Geschwindigkeiten von 600 m/s und mehr.

Im Rahmen dieser Erfindung wird daher unter "Impuls(übertragung)" auch synonym eine Kraft-, Druckübertragung oder allgemein Beaufschlagung verstanden, welche jedenfalls ausreichend ist, die auf der hautseitigen Membran (15) aufgebrachten Substanzen (25) auf die gewünschten hohen Geschwindigkeiten zu beschleunigen und abzulösen, wodurch sie zunächst frei in Richtung des Gewebes bzw. Haut (18) oder Körper fliegen, um dort einzuschlagen, die Gewebe oder Körperbarriere durchbrechen und in die gewünschte Tiefe des Gewebes, insbesondere der Haut oder des Körpers eindringen.

Im Rahmen dieser Erfindung bedeutet "Haut" eine Gewebebarriere eines Menschen, Säugetier oder Tier nach außen zur Umwelt hin. Die Haut übt zahlreiche lebenswichtige Funktionen aus, insbesondere die Epidermis, ganz besonders das "stratum corneum, die außen liegende Schicht der Epidermis, dient als Barriereorgan. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedene polare Lipide werden im Keratinisierungsprozess freigesetzt. Durch die erfindungsgemäße nadellose oder nadelfreie Applikation können infolge dessen die Stoffe in den Körper lokal eintreten und ggfs. danach auch in die Blutbahnen und/oder in die Lymphbahnen eintreten.

Im Rahmen dieser Erfindung bedeutet "aufgebrachte Substanzen oder Substanzauftragung (25)", solche, die zumindest einen Stoff enthalten und beispielsweise mit Ölen u.a. an der äußeren (hautseitigen) Membran (15) fixiert sind oder durch Adhäsion anhaften. Beispielsweise können die aufgebrachten Substanzen Mittel enthalten, die ein Trocknen des Wirkstoffes auf der besagten hautseitigen Membran erlauben. Insbesondere sind zusätzliche Haftvermittler oder Additive geeignet und beizufügen, wie Öle, Kohlehydratlösungen, Aminosäurelösungen, Polymerlösungen, oder andere vorzugsweise pharmazeutisch geeignete Hilfs- und Zusatzstoffe.

Der Begriff "Substanzen" umfasst sämtliche Mittel und Stoffe, die zur zweckmäßigen Auftragung auf die Membran geeignet sind (z.B. Pulver, Partikel etc.), einschließlich ein oder mehrerer Wirkstoffe (z.B. Arzneimittel). Die Substanzen werden auf die hautseitige Membran (15) aufgebracht und sind vor der Membran der Haut direkt gegenüberliegend ausgerichtet. Es erfolgt keine Substanzauftragung in Richtung der Brennkammer (11) und auf der brennkammerseitigen Membran (13).

Die erforderliche Aktivierungsenergie zur Impulsübertragung kann mittels einer Aktivierungseinheit erfolgen und zwar mittels Anzünder oder Zündstift und über einen Auslösungsmechanismus mittels Reibung, Stoßen oder geeigneter Stromversorgung.

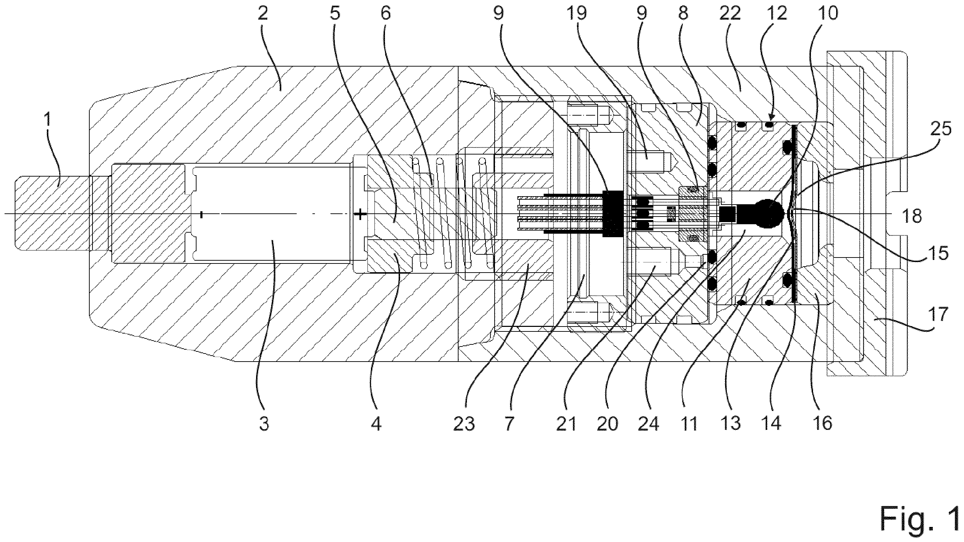

Eine geeignete Aktivierungseinheit ist beispielsweise in Figur 1 dargestellt. Mittels eines Auslösers oder Tasters (1) wird die Batterie (3) gegen eine Kontaktfeder (6) und gegen einen Kontaktstift (5) gedrückt, dadurch der Stromkreis über die Anschlüsse (9) und das EED (10) geschlossen und infolge die Zündung des EEDs (10) herbeigeführt. Alternative Auslösungsmechanismen sind dem Fachmann bekannt (Reibdraht, Schlagdraht, Knackfrosch auf stoßempfindliche Anzündmischung schlagend etc.).

In einer weiteren bevorzugten Ausführungsform ist die besagte Aktivierungseinheit (1, 2, 3, 4, 5, 6, 7, 8, 9, 10) in axialer Richtung zur Kammer (11) samt Verbrennungsraum (24) ausgerichtet und der Membran (15) gegenüberliegend.

Die Membran (15) kann fixiert oder beweglich bzw. verschiebbar sein und ebenfalls ist als Doppelmembran (13, 15) bzw. Mehrschichtmembran ausgestaltet und folglich ist eine solche Membran erfindungsgemäß der Adressat einer solchen Impulsübertragung, so dass eine entsprechende Beschleunigung der Substanz aus einer Substanzauftragung (25) der hautseitigen Membran (15) in Richtung Gewebe entstehen kann.

In einer bevorzugten Ausführungsform besteht die erfindungsgemäße Membran (15) aus Metall oder einem Material entsprechender Härte und Duktilität, solche wie Stahl, Kunststoffen, besonders bevorzugt jedoch Titan oder Titanblech. Insbesondere Titan weist eine vorteilhafte hohe Ziehgrenze bei sehr guter Beschleunigbarkeit durch seine geringere spezifische Dichte gegenüber Stahl bei gleich hoher mechanischer Belastbarkeit und besserer Zähigkeit auf, wodurch schon bei relativ kleinen Drücken in der Brennkammer eine deutlich höhere Geschwindigkeit der Membranoberfläche bzw. der ausbeulenden Membran erreicht wird. Hier maßgeblich ist die Beschleunigungskennzahl BK=Fließgrenze sigma0,2 / Dichte ro.

Anstelle einer Einfachmembran ist eine Doppelmembran vorgesehen d.h. beispielsweise statt einer 1 mm starken Einfachmembran werden zwei Membranen mit jeweils 0,2 oder 0,3 oder 0,4 oder 0,5 mm übereinandergelegt, um eine Doppelmembran auszubilden. Ebenfalls andere Stärkeverhältnisse sind möglich, wie beispielsweise 0,6 mm Richtung Kammer und 0,4 mm Richtung Haut oder 0,2, und 0,3 mm oder 0,3 und 0,4 oder 0,4 und 0,5 oder 0,2 und 0,6 usw..

Eine Mehrschichtmembran liegt erfindungsgemäß vor, wenn mehr als 2 Schichten übereinandergelegt werden. So kann beispielsweise zwischen der Doppelmembran eine randnahe Dichtschicht oder eine Dämmschicht eingebracht sein, um die Geschwindigkeitsverteilung der ablösenden Teilchen zu verändern.

Eine Doppelmembran vereint hierbei bei gleicher Gesamtstärke der Membran die Vorteile einer besseren Flexibiliät bzw. Verformbarkeit mit einer besseren Sicherheit für den Fall, dass eine Membran einen Mikroriß aufweist oder während der Verformung erhält. Auch in diesen Fällen würde ein Austreten des hochgespannten Gases und der bedrückten Inhaltsstoffe aus der Brennkammer Richtung Gewebe bzw. Haut mit an Sicherheit grenzender Wahrscheinlichkeit verhindert werden.

Im Fall einer fixierten Doppelmembran (13, 15) sind beide Membranen - die Doppelmembran - vor einer Stützscheibe (16), insbesondere an die Kammer (11), angebracht / fixiert, wobei sie im Randbereich etwas beweglich ist, also leicht während der Verformung etwas Richtung Zentrum gezogen werden kann. Weiterhin ist bevorzugt, dass die nach innen gerichtete brennkammerseitige Membran (13) eine Dicke von 0,1 mm bis 0,6 mm aufweist, vorzugsweise 0,2 mm bis 0,6 mm, besonders bevorzugt 0,5 mm. Die nach außen gerichtete hautseitige Membran (15) weist vorzugsweise ebenfalls eine Dicke von 0,1 mm bis 0,6 mm auf, vorzugsweise 0,2 mm bis 0,6 mm, besonders bevorzugt 0,3mm bis 0,5 mm. Die Membranen (13, 15) können sich ganz oder teilweise berühren.

In einer weiteren bevorzugten Ausführungsform weist die innere Membran (13) zur hautseitigen Membran (15) einen Abstand auf und zwar vorzugsweise von 0,2 mm bis 1,5 mm, besonders bevorzugt 1 mm. Der erforderliche Abstand kann mittels eines Abstandhalters (14) zwischen den beiden Membranen ausgelegt werden. Diese Maßnahme kann eine effiziente Verhinderung des Platzens der hautseitigen Membran bedeuten.

In einer weiteren besonders bevorzugten Ausführungsform ist die Membran oder Doppelmembran (13, 15) zur Kammer (11) und in dessen Verbrennungsraum (24) hin ganz oder teilweise ausgewölbt, ganzflächig oder vorzugsweise im zentralen radialen Bereich um den Membranmittelpunkt. Eine solche jeweils konvexe Wölbung der jeweiligen Membranen aus Sicht der Brennkammer kann der Fachmann mittels bekannter Methoden erreichen und bewirkt eine höhere Beschleunigung der Membranoberfläche.

In einer weiteren bevorzugten Ausführungsform liegen die beiden Membranen (13, 15) an einer Stützscheibe (16) an. Diese Stützscheibe ist vorzugsweise aus einem stoßunempfindlichen und gut stoßdämpfenden Kunststoff wie beispielsweise POM (=Delrin) oder Metall, beispielsweise Messing hergestellt, und hat die Aufgabe, ein Platzen der Membran durch den nach der Beschleunigung der Membranoberfläche weiter in der Brennkammer bzw. an der Membran anliegenden hohen Druck zu verhindern.

Gleichzeitig kann die Stützscheibe (16) an seiner Innenkontur mit einem weichen Kunststoff dünn überzogen sein, z.B. mit Polyethylen, um das Auslösegeräusch der Vorrichtung vorteilhaft deutlich zu reduzieren - es wird beispielsweise das knallartige Geräusch vermieden, welches entsteht, wenn eine Metallmembran auf die Oberfläche einer metallenen Stützscheibe aufschlägt.

Die vorgenannten Ausführungsformen einer Membran oder Doppelmembran erlauben eine optimierte Impulsübertragung (supra).

Ein grundlegender und gattungsgemäßer Aufbau einer nadellosen Injektionsvorrichtung mit fixierter Membran ist z.B. in Figur 1 samt Legende gezeigt, wobei für die erfindungsgemäße Funktion der Injektionsvorrichtung das Gehäuse (22), sowie die Teile (7) bis (16) zu berücksichtigen sind. Die Teile (7) bis (16) können hierbei jederzeit in ein anders geformtes Gehäuse (22) eingebaut und ein EED, insbesondere ein Detonator (10), auch anders mit Energie versorgt werden, ohne die Funktion der Teile bzw. der Vorrichtung zu verändern. Besondere Ausführungsformen werden näher beschrieben.

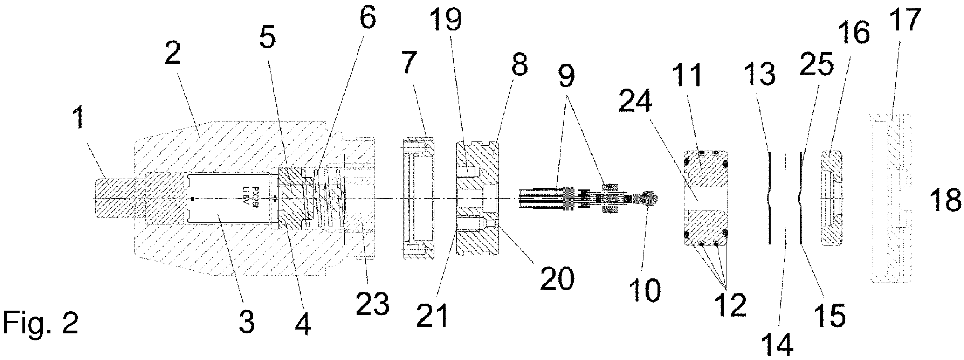

Die Geometrie der einzelnen Teile in Figur 1 können je nach Anforderung geändert oder teilweise integral zusammengefasst werden. Beispielsweise können Teile (7) bis (9) zu einem Teil integral zusammengefasst werden, oder Teil (7) kann entfallen, falls Teil (8) beispielsweise ein Außengewinde aufweist und in das Gehäuse (22) eingeschraubt wird - oder beispielsweise per Sprengring im Gehäuse (22) gehalten wird - oder ein Einlegeteil in der Spritzform des Gehäuses (22) ist (siehe ebenfalls Figur 2). Ebenfalls können die Teile (1), sowie die Teile (4) bis (6) und (23) durch die Verwendung eines herkömmlichen Tasters zur elektrischen Kontaktierung des Detonators (EED) (10) mit der Batterie (3) entfallen.

Die Kammer (11) weist vorzugsweise O-Ringe zur Abdichtung (12) auf. Weiterhin kann die Kammer (11) eine Entlüftung (20) samt zugehöriger Abdeckung (21) aufweisen.

In einer weiteren bevorzugten Ausführungsform enthält die Injektionsvorrichtung einen Aufsatz (17) und / oder Stützscheibe (16), so dass zwischen der Haut (18) und der Membran (15) ein definierter Abstand von beispielsweise ca. 5-10 mm erreicht wird (supra), um die sich auswölbende Membran sicher von der Haut bzw. der Oberfläche des Körpers entfernt zu halten, als auch eine definierte Flugstrecke der Substanzen zu erhalten, wobei die Membran vorzugsweise parallel zur Haut beabstandet ist.

Weiterhin betrifft die Erfindung die Verwendung oder Anwendung von ein oder mehreren Substanzen, insbesondere Stoffe, Wirkstoffe, Arzneimittel zur nadelfreien Applikation mittels der erfindungsgemäßen Injektionsvorrichtung nach einer der vorstehenden Ausführungsformen.

Weiterhin betrifft die Erfindung die Verwendung oder Anwendung von Substanzen, insbesondere Stoffen mit beliebiger Funktion und Eignung, solche wie Farb- oder Schmierstoffe oder Materialien mit anderen besonderen Eigenschaften zur nadellosen Einbringung in bzw. unter die Oberflächen oder Körper technischer Gegenstände mittels der erfindungsgemäßen Injektionsvorrichtung nach einer der vorstehenden Ausführungsformen.

Daher betrifft die Erfindung eine Injektionsvorrichtung nach einer der vorstehenden Ausführungsformen zur Verwendung einer Substanz, eines Stoffes oder eines Wirkstoffes, insbesondere Arzneimittel, zur nadelfreien Applikation.

Die folgenden Beispiele und Figuren sollen die Erfindung näher erläutern, ohne jedoch diese zu beschränken.

Figur 1 zeigt einen Querschnitt der erfindungsgemäßen Injektionsvorrichtung mit fixierter Membran.

Figur 2 zeigt die Einzelbestandteile der erfindungsgemäßen Injektionsvorrichtung.

### Beispiel 1:

Herstellung von blasenfreien und luftarmen Silikonoleogelen zur direkten Befüllung der erfindungsgemäßen nadellosen Injektionsvorrichtung.

### Silikonöle

- Silikonöl (100 cSt) von Sigma-Aldrich (Sigma-Aldrich, Taufkirchen, Germany)
- Silikonöl (500 cSt) von Sigma-Aldrich (Sigma-Aldrich, Taufkirchen, Germany)
- Silikonöl (1000 cSt) von Sigma-Aldrich (Sigma-Aldrich, Taufkirchen, Germany)

### Hochdisperses Siliziumdioxid

- Aerosil R200 Pharma (Evonik, Hanau-Wolfgang, Germany)
- Aerosil R972 Pharma (Evonik, Hanau-Wolfgang, Germany)
- Luer-Spritze (Norm-Ject) (Henke Sass Wolf, Tuttlingen, Germany)
- Luer-Adapter (Combifix Adapter) (B. Braun, Melsungen, Germany)
- Luer-Stopper (Combi-Stopper) (B. Braun, Melsungen, Germany)
- Vakuumtrockenschrank (Memmert, Schwabach, Germany)

### Methode:

Der Kolben einer Luer-Spritze wird entnommen und die Luer-Öffnung wird mit einem Luer- Stopper verschlossen. In die Luer-Spritze wird die abgewogene Menge Aerosil von der Öffnung (kolbenseitig) eingefügt. Es werden abhängig von der angestrebten Konzentration verschiedene Mengen an Aerosil eingewogen: (4% (m/m), 5% (m/m), 6% (m/m), 10% (m/m). Der Kolben der Spritze wird vorsichtig wieder in die Luer-Lock-Spritze eingesetzt, ohne das Aerosil stark zu komprimieren. Der Luer-Stopper wird entfernt und durch einen Luer-Adapter ersetzt. Eine weitere Luer-Spritze wird, mit zuvor entferntem Kolben, mit dem Luer-Adapter verbunden. Die zwei verbundenen Spritzen werden auf einem Träger befestigt. Die abgewogene Menge an Silikonöl (m/m) wird in die zweite, leere Luer-Spritze eingegossen. Somit ist in der unteren Luer-Spritze das Aerosil mit dem Luer-Adapter mit dem Silikonöl in der oberen Luer-Spritze verbunden.

Die verbundenen und befüllten Spritzen werden in einen Vakuumtrockenschrank eingesetzt und mindestens 4 Stunden bei 10 mbar Druck entlüftet. Dabei entweicht die Luft aus dem Aerosil und dem Silikonoel. Durch das Angleichen an Normalatmosphäre wird in die luftarme und aerosilhaltige untere Luer-Spritzenkammer das Silikonöl gedrückt. Das Silikonöl vermischt sich mit dem Aerosil und verhindert gleichzeitig das Eindringen von Luft in die Mischung. Die leere obere Luer-Spritze wird entfernt. Eine neue Luer-Spritze wird an den Luer-Adapter angebunden. Jedoch muss darauf geachtet werden, dass der Luer-Adapter bis zum Rand mit der viskosen Mischung gefüllt ist, um Lufteinschlüsse durch die Verbindung der neuen Spritze zu vermeiden. Das Silikonöl-Aerosil-Gemisch wird zur Homogenisierung 20-mal durch den Luer-Adapter in die angebundene Spritze gedrückt. Anschließend werden Luer-Spritze und Luer-Adapter entfernt und das entstandene Silikonoleogel wird mit einem Luer-Stopper in der Spritze verschlossen. Das Silikonolegel ist nach dem Vermischen direkt für die Anwendung nutzbar. Das in der Spritze befindliche Gel wird über den Luer Konus in das erfindungsgemäße Device bzw. die dort befindliche Brennkammer eindosiert, indem der Kolben entsprechend der üblichen Funktion der Spritze bewegt wird.

### Beispiel 2:

### Bestimmung der Viskosität der Silikonoleogele

### Materialien

- Silkonoelgele werden gemäß der Vorschrift nach Beispiel 1 hergestellt.- 4%, 5%, 6% Silikonoleogele mit Aerosil R200 Pharma
- 4%, 5%, 6%, 10% Silikonoleogele mit Aerosil R972 Pharma

### Gerätschaften

- Rheomether MCR100 von Anton Paar (Anton Paar, Graz, Austria)
- Platte PP50 (Diameter: 49.958 mm)
- Arbeitsspalt 500 µm
- Messtemperatur: 250C
- Messverfahren: Ansteigende Scherrate von 1-100 s-1

Eine ausreichende Menge des zu untersuchenden Oleogels wird auf die Arbeitsfläche des Rheometers aufgetragen. Die Plattenhöhe wird justiert und austretendes, überflüssiges Oleogel wird vorsichtig entfernt. Eine Messung mit ansteigender Scherrate wird durchgeführt. - Bei einer Scherrate von 36 s⁻¹ wurden folgende Viskositäten ermittelt:

**Tabelle 1: Viskositäten der Silikonoleogele bei einer Scherrate von 36 s⁻¹**

| Silikonoleogel | | Viskosität [Pa s] |
|---|---|---|
| 4% Silikonoleogel | (R200 Pharma) | 8.3 ± 0.43 |
| 5% Silikonoleogel | (R200 Pharma) | 13.5 + 0.68 |
| 6% Silikonoleogel | (R200 Pharma) | 19.2 + 1.65 |
| 4% Silikonoleogel | (R972 Pharma) | 303.5 + 8.7 |
| 5% Silikonoleogel | (R972 Pharma) | 450.7 + 37.9 |
| 6% Silikonoleogel | (R972 Pharma) | 560.7 + 38.4 |
| 10% Silikonoleogel | (R972 Pharma) | 1645.0 + 161.3 |

### Legende:

- 1: Auslöser, Taster
- 2: Auslösergehäuse
- 3: Batterie
- 4: Federkappe
- 5: Kontaktstift
- 6: Kontaktfeder
- 7: Nutmutter
- 8: Halter für Glasdurchführung
- 9: Durchführung der Anschlüsse des EED
- 10: EED (Detonator)
- 11: Brennkammer oder Kammer
- 12: O-Ring-Abdichtung der Brennkammer (kann bei einigen Ausführungsformen der Vorrichtung auch entfallen)
- 13: Innere oder brennkammerseitige Membran
- 14: Abstandshalter, übernimmt auch Abdichtfunktionen
- 15: Hautseitige Membran, fixiert zu Membran (13) und Abstandshalter (14)
- 16: Stützscheibe
- 17: Aufsatz
- 18: Haut oder die zu penetrierende Oberfläche (liegt auf 17 an, nicht gezeichnet)
- 19: Gewinde für Masseanschlussschraube (kann bei einigen Ausführungsformen der Vorrichtung auch entfallen)
- 20: Brennkammerentlüftung
- 21: Abdeckung, Entlüftung
- 22: Gehäuse für die Teile 7-21
- 23: Führung für Kontaktstift
- 24: Brennkammerraum oder Verbrennungsraum (24) enthaltend einen pyrotechnischen Stoff und einen gelartigen Stoff
- 25: Substanzauftragung in Richtung des Gewebe bzw. Haut

## Patentansprüche

1. Nadellose Injektionsvorrichtung umfassend eine Brennkammer (11) mit einem Verbrennungsraum (24), wobei die Brennkammer (11) an einer Austrittsöffnung mindestens eine Membran (15) aufweist, und mit einer Substanzauftragung (25) versehen ist, wobei die Membran als Doppelmembran ausgebildet ist, bestehend aus einer brennkammerseitigen Membran (13) und einer hautseitigen Membran (15) und die brennkammerseitige Membran (13) in den Verbrennungsraum (24) mündet, **dadurch gekennzeichnet,**
**dass** die Brennkammer (11) a) mindestens ein pyrotechnisches Material und b) mindestens einen gelartigen Stoff ausgewählt aus einem Gel, oder einem Gelbildner und
einer Flüssigkeit, in einem Verbrennungsraum (24) enthält und die brennkammerseitige Membran (13) zur hautseitigen Membran (15) einen Abstand von 0,2 mm bis 1,0 mm oder 1,5 mm aufweist und der Abstand mittels eines Abstandhalters (14) zwischen der brennkammerseitigen Membran (13) und der hautseitigen Membran (15) ausgelegt wird, sodass eine hinreichend schnelle Ausbauchung oder eine ausreichend große Impulsübertragung auf die hautseitige Membran (15) erfolgt.

2. Nadellose Injektionsvorrichtung gemäß Anspruch 1, wobei der gelartige Stoff ein Gel ist, ausgewählt aus der Gruppe Oleogel, Lipogel, Paraffingel, Silikonoleogel.

3. Nadellose Injektionsvorrichtung gemäß Anspruch 1 oder Anspruch 2, wobei der gelartige Stoff gasfrei ist.

4. Nadellose Injektionsvorrichtung nach einem der vorstehenden Ansprüche, wobei der gelartige Stoff aus einem Gelbildner und einer hydrophoben Flüssigkeit besteht.

5. Nadellose Injektionsvorrichtung nach einem der vorstehenden Ansprüche, wobei der Gelbildner ein organischer oder anorganischer Gelbildner ist, ausgewählt aus der Gruppe Pektin, Tragant, Polyacrylsäuren, Polyvinylpyrrolidon, Siliciumdioxid, hochdisperses Siliciumdioxid, Carboxymethylcellulose, Carbomere (Polyacrylsäure), Celluloseether, Poloxamere.

6. Nadellose Injektionsvorrichtung gemäß Anspruch 4, wobei die hydrophobe Flüssigkeit ausgewählt ist aus der Gruppe pflanzliche oder tierische Öle, Triglyceride, Mono- und Diglyceride, Phospholipide, flüssige Wachse, Alkohole, Silikonöle, Paraffine.

7. Nadellose Injektionsvorrichtung nach einem der vorstehenden Ansprüche, wobei die Kammer (11) vollständig mit einem gelartigen Stoff (24) befüllt ist.

8. Nadellose Injektionsvorrichtung nach einem der vorstehenden Ansprüche, wobei die hautseitige Membran (15) aus Stahl, Kunststoffen, Titan oder Titanblech besteht.

9. Nadellose Injektionsvorrichtung nach einem der vorstehenden Ansprüche, wobei die hautseitige Membran (15) eine Dicke von 0,1 mm bis 0,6 mm aufweist.

10. Nadellose Injektionsvorrichtung nach einem der vorstehenden Ansprüche, wobei die brennseitige Membran (13) und hautseitige Membran (15) vor einer Stützscheibe (16) an die Brennkammer (11) fixiert sind.

11. Nadellose Injektionsvorrichtung nach Anspruch 10, wobei die Stützscheibe (16) aus einem stoßdämpfenden Kunststoff oder Metall besteht.

12. Nadellose Injektionsvorrichtung nach einem der vorstehenden Ansprüche, wobei die brennkammerseitige Membran (13) oder hautseitige Membran (15) gewölbt ist.

13. Nadellose Injektionsvorrichtung nach einem der vorstehenden Ansprüche, enthaltend einen Aufsatz (17) und / oder Stützscheibe (16), wobei zwischen der Haut (18) und der hautseitigen Membran (15) ein Abstand von mindestens 5 mm erreicht wird.

14. Nadellose Injektionsvorrichtung nach einem der vorstehenden Ansprüche, wobei eine Aktivierungseinheit (1, 2, 3, 4, 5, 6, 7, 8, 9, 10) in axialer Richtung zur hautseitigen Membran (15) vorhanden ist.

15. Nadellose Injektionsvorrichtung nach einem der vorstehenden Ansprüche zur Verwendung eines Stoffes oder eines Wirkstoffes oder Arzneimittels zur nadelfreien Applikation.

## Claims

1. A needleless injection device, comprising a firing chamber (11) including a combustion chamber (24), the firing chamber (11) comprising at least one membrane (15) at a discharge opening, and being provided with a substance application (25), the membrane being formed as a double membrane, composed of a firing chamber-side membrane (13) and a skin-side membrane (15), and the firing chamber-side membrane (13) leading into the combustion chamber (24), **characterized in that**
the firing chamber (11) comprises a) at least one pyrotechnic material and b) at least one gel-like medium selected from a gel, or a gelling agent and a liquid, in a combustion chamber (24), and the firing chamber-side membrane (13) has a distance from the skin-side membrane (15) of 0.2 mm to 1.0 mm or 1.5 mm, and the distance is formed, by means of a spacer (14) between the firing chamber-side membrane (13) and the skin-side membrane (15), so that sufficiently rapid bulging or a sufficiently large pulse transfer to the skin-side membrane (15) occurs.

2. The needleless injection device according to claim 1, wherein the gel-like medium is a gel selected from the group consisting of oleogel, lipogel, paraffin gel, and silicone oleogel.

3. The needleless injection device according to claim 1 or claim 2, wherein the gel-like medium is gas-free.

4. A needleless injection device according to any one of the preceding claims, wherein the gel-like medium is composed of a gelling agent and a hydrophobic liquid.

5. A needleless injection device according to any one of the preceding claims, wherein the gelling agent is an organic or inorganic gelling agent, selected from the group consisting of pectin, tragacanth, polyacrylic acids, polyvinylpyrrolidone, silicon dioxide, highly dispersed silicon dioxide, carboxymethyl cellulose, carbomers (polyacrylic acid), cellulose ethers, and poloxamers.

6. The needleless injection device according to claim 4, wherein the hydrophobic liquid is selected from the group consisting of vegetable or animal oils, triglycerides, monoglycerides and diglycerides, phospholipids, liquid waxes, alcohols, silicone oils, and paraffins.

7. A needleless injection device according to any one of the preceding claims, wherein the chamber (11) is completely filled with a gel-like medium (24).

8. A needleless injection device according to any one of the preceding claims, wherein the skin-side membrane (15) is made of steel, plastic materials, titanium, or sheet titanium.

9. A needleless injection device according to any one of the preceding claims, wherein the skin-side membrane (15) has a thickness of 0.1 mm to 0.6 mm.

10. A needleless injection device according to any one of the preceding claims, wherein the firing chamber-side membrane (13) and the skin-side membrane (15) are fixed to the firing chamber (11) in front of a support disk (16).

11. The needleless injection device according to claim 10, wherein the support disc (16) is composed of a shock-absorbing plastic material or metal.

12. A needleless injection device according to any one of the preceding claims, wherein the firing chamber-side membrane (13) or the skin-side membrane (15) is curved.

13. A needleless injection device according to any one of the preceding claims, comprising an attachment (17) and/or a support disc (16), a distance of at least 5 mm being achieved between the skin (18) and the skin-side membrane (15)

14. A needleless injection device according to any one of the preceding claims, wherein an activation unit (1, 2, 3, 4, 5, 6, 7, 8, 9, 10) is present in the axial direction relative to the skin-side membrane (15).

15. A needleless injection device according to any one of the preceding claims for use of a medium or an active substance or medicinal product for needle-free application.

## Revendications

1. Dispositif d'injection sans aiguille comprenant une chambre de combustion (11) dotée d'un espace de combustion (24), où la chambre de combustion (11) présente au niveau d'un orifice de sortie au moins une membrane (15), et est pourvue d'un revêtement avec une substance (25), où la membrane est conçue sous forme de membrane double, constituée d'une membrane côté chambre de combustion (13) et d'une membrane côté peau (15) et la membrane côté chambre de combustion (13) débouche dans l'espace de combustion (24), **caractérisé en ce**
**que** la chambre de combustion (11) contient a) au moins un matériau pyrotechnique et b) au moins une substance de type gel choisie parmi un gel, ou un agent filmogène et un liquide dans un espace de combustion (24), et la membrane côté chambre de combustion (13) présente par rapport à la membrane côté peau (15) une distance de 0,2 mm à 1,0 mm ou 1,5 mm, et la distance est mesurée au moyen d'un espaceur (14) entre la membrane côté chambre de combustion (13) et la membrane côté peau (15) de sorte qu'il se produit un renflement suffisamment rapide ou un transfert d'impulsion suffisamment important sur la membrane côté peau (15).

2. Dispositif d'injection sans aiguille selon la revendication 1, dans lequel la substance de type gel est un gel choisi dans le groupe d'un oléogel, d'un lipogel, d'un gel de paraffine, d'un oléogel de silicone.

3. Dispositif d'injection sans aiguille selon la revendication 1 ou la revendication 2, dans lequel la substance de type gel est exempte de gaz.

4. Dispositif d'injection sans aiguille selon l'une des revendications précédentes, dans lequel la substance de type gel est constituée d'un agent filmogène et d'un liquide hydrophobe.

5. Dispositif d'injection sans aiguille selon l'une des revendications précédentes, dans lequel l'agent filmogène est un agent filmogène organique ou inorganique, choisi dans le groupe de la pectine, de l'adragante, des acides poly acryliques, de la polyvinylpyrrolidone, du dioxyde de silicium, du dioxyde de silicium hautement dispersé, de la carboxyméthyl cellulose, des carbomères (acides polyacrylique), des éthers de cellulose, des poloxamères.

6. Dispositif d'injection sans aiguille selon la revendication 4, dans lequel le liquide hydrophobe est choisi dans le groupe des huiles végétales ou animales, des triglycérides, des mono et diglycérides, des phospholipides, des cires liquides, des alcools, des huiles de silicone, des paraffines.

7. Dispositif d'injection sans aiguille selon l'une des revendications précédentes, dans lequel la chambre (11) est totalement remplie avec une substance de type gel (24).

8. Dispositif d'injection sans aiguille selon l'une des revendications précédentes, dans lequel la membrane côté peau (15) est constituée d'acier, de matières plastiques, de titane ou de tôle de titane.

9. Dispositif d'injection sans aiguille selon l'une des revendications précédentes, dans lequel la membrane côté peau (15) présente une épaisseur de 0,1 mm à 0,6 mm.

10. Dispositif d'injection sans aiguille selon l'une des revendications précédentes, dans lequel la membrane côté combustion (13) et la membrane côté peau (15) sont fixées sur la chambre de combustion (11) devant un disque de support (16).

11. Dispositif d'injection sans aiguille selon la revendication 10, dans lequel le disque de support (16) est constitué d'une matière plastique ou d'un métal amortissant les coups.

12. Dispositif d'injection sans aiguille selon l'une des revendications précédentes, dans lequel la membrane côté chambre de combustion (13), ou la membrane côté peau (15), est courbe.

13. Dispositif d'injection sans aiguille selon l'une des revendications précédentes, contenant un caisson (17) et/ou un disque de support (16), où une distance d'au moins 5 mm est atteinte entre la peau (18) et la membrane côté peau (15).

14. Dispositif d'injection sans aiguille selon l'une des revendications précédentes, dans lequel il y a une unité d'activation (1, 2, 3, 4, 5, 6, 7, 8, 9, 10) dans la direction axiale par rapport à la membrane côté peau (15).

15. Dispositif d'injection sans aiguille selon l'une des revendications précédentes, destinée à l'utilisation d'une substance, ou d'une substance active, ou d'un médicament pour une application sans aiguille.
